# EUROPÄISCHE PATENTSCHRIFT

(11) **EP 3 988 483 B1**
(45) Veröffentlichungstag und Bekanntmachung des Hinweises auf die Patenterteilung: **03.01.2024**
(21) Anmeldenummer: 20203800.6
(22) Anmeldetag: 26.10.2020
(51) Int. Cl.: B65H 18/00, A61F 15/00, B65H 18/08, B65H 23/10

(54) **VORRICHTUNG ZUM HANDHABEN VON BANDFÖRMIGEN MATERIALBAHNEN, INSBESONDERE VON BINDEN**
DEVICE FOR HANDLING STRIPS OF MATERIAL, IN PARTICULAR BANDAGES
DISPOSITIF DE MANUTENTION DES NAPPES DE MATIÈRE EN FORME DE BANDE, EN PARTICULIER DES BANDEAUX

(43) Veröffentlichungstag der Anmeldung: 27.04.2022
(73) Patentinhaber: BK Grundbesitz GmbH & Co. KG, 78586 Deilingen (DE)
(72) Erfinder: KRAFT, Oliver, 78586 Deilingen (DE); BOZIC, Roman, 72367 Weilen ud.d.R. (DE)
(74) Vertreter: Westphal, Mussgnug & Partner Patentanwälte mbB

(56) Entgegenhaltungen:
- CN-U- 209 499 827
- DE-A1-102009 042 538
- DE-U1- 8 702 414

## Beschreibung

Die vorliegende Erfindung betrifft eine Vorrichtung zum Handhaben von bandförmigen Materialbahnen, insbesondere von Binden, gemäß den Merkmalen des Oberbegriffs des Anspruchs 1. Eine solche Vorrichtung ist z B auf DE 10 2009 042 538 A1 bekannt, die die Merkmale des Oberbegriffs des Anspruchs 1 offenbart.

In medizinischen Einrichtungen wie Krankenhäusern und Altenheimen, im Sportbereich sowie im privaten Umfeld werden als sogenannte elastische Binden ausgebildete bandförmige Materialbahnen, auch als Verbandsbinden bezeichnet, für vielfältige medizinische Zwecke verwendet, beispielsweise als Stützverbände. Hierbei werden die Binden insbesondere um den Arm oder um das Bein einer Person gewickelt.

Derartige Binden werden beim Anlegen oftmals manuell von einer Rolle abgewickelt, wobei die Spannung, mit der die Binde beim Anlegen beaufschlagt wird, durch den Benutzer individuell vorgegeben wird. Soll eine benutzte Binde wiederverwendet werden, wird die abgenommene Binde typischerweise gereinigt, desinfiziert und in Vorbereitung des erneuten Anlegens manuell aufgewickelt, insbesondere deshalb, um sie platzsparend lagern und transportieren zu können. Das manuelle Aufwickeln erfordert eine gewisse Geschicklichkeit und ist zeitaufwendig. Da das Anlegen in den meisten Fällen von Pflegediensten vorgenommen wird, die wirtschaftlich arbeiten müssen, stellt das manuelle Aufwickeln ein Hindernis für den wirtschaftlichen Einsatz derartiger Binden dar.

Um eine Abhilfe für diese Situation zu schaffen, sind Vorrichtungen zum Handhaben von bandförmigen Materialbahnen, insbesondere von Binden, entwickelt worden, von denen einige Ausführungen beispielsweise in der oben genannten DE 10 2009 042 538 A1 und der EP 3 473 227 A1 offenbart sind. Bei der in der DE 10 2009 042 538 A1 offenbarten Vorrichtung wird die Binde zunächst über eine Anzahl von konvex geformten Transportrollen geführt, bevor die Binde auf einen Wickeldorn aufgewickelt wird. Hierdurch baut die Vorrichtung vergleichsweise groß und wird relativ schwer. Zudem wird die Herstellung der Vorrichtung aufgrund der Notwendigkeit der Lagerung der Transportrollen verkompliziert.

Die in der EP 3 473 227 A1 offenbarte Vorrichtung weist einen Vorratsbehälter auf, mit welchem eine Substanz beim Aufwickeln auf die Binde aufgebracht werden kann, insbesondere eine Substanz zum Desinfizieren der Binde. Auch hierdurch baut die Vorrichtung vergleichsweise groß.

Sowohl die Vorrichtung gemäß der DE 10 2009 042 538 A1 als auch diejenige der EP 3 473 227 A1 eignen sich daher nur bedingt für den täglichen Gebrauch. Zudem wird die Binde in beiden Fällen insbesondere dann, wenn die Binde zerknittert ist, ungleichmäßig aufgewickelt, so dass sie sich schlecht lagern lässt.

Aufgabe einer Ausführungsform der vorliegenden Erfindung ist es, eine Vorrichtung zum Handhaben von bandförmigen Materialbahnen, insbesondere von Binden vorzuschlagen, mit welcher es mit einfachen und kostengünstigen Mitteln möglich ist, gebrauchte Binden schnell und gleichmäßig aufzuwickeln.

Diese Aufgabe wird mit den in Anspruch 1 angegebenen Merkmalen gelöst. Vorteilhafte Ausführungsformen sind Gegenstand der Unteransprüche.

Eine Ausführungsform der Erfindung betrifft eine Vorrichtung zum Handhaben von bandförmigen Materialbahnen, insbesondere von Binden, umfassend
- ein Gehäuse mit einer ersten Gehäusehälfte und einer zweiten Gehäusehälfte, die einen Hohlraum umschließen und zwischen einer Offenstellung und einer Schließstellung drehbar miteinander verbindbar oder verbunden sind, wobei in der Schließstellung das Gehäuse einen spaltförmigen Durchtrittsabschnitt bildet, durch welchen der Hohlraum zugänglich ist, und
- eine im Gehäuse drehbar gelagerte Wickeleinheit, mit welcher eine Materialbahn durch den Durchtrittsabschnitt in den Hohlraum einziehbar ist und aufwickelbar ist, wobei
- im Durchtrittsabschnitt mit der Materialbahn beim Einziehen zusammenwirkende Streckmittel zum Aufbringen einer Materialspannung angeordnet sind.

Streckmittel, mit denen eine Materialspannung auf die Materialbahn aufgebracht werden kann, können beispielsweise federgelagerte Kugeln umfassen, wobei die Kugeln mit der Materialbahn zusammenwirken. Aufgrund der Tatsache, dass die im Durchtrittsabschnitt angeordneten Streckmittel eine Materialspannung auf die Materialbahn aufbringen, wird die Materialbahn beim Aufwickeln vorgespannt, wodurch sie sich sehr gleichmäßig aufwickeln lässt. Zwar bewirken die Transportrollen der Vorrichtung, welche aus der DE 10 2009 042 538 A1 bekannt sind, auch eine Vorspannung der Materialbahn, allerdings sind die Transportrollen nicht im Durchtrittsabschnitt des Gehäuses angeordnet, sondern befinden sich im Hohlraum, wodurch die Vorrichtung größer baut als die vorschlagsgemäße Vorrichtung. Im Gegensatz dazu ermöglicht die Anordnung der Streckmittel im Durchtrittsabschnitt eine einfache und kompakte Gestaltung der vorschlagsgemäßen Vorrichtung.

Nach Maßgabe einer weiteren Ausführungsform umfassen die Streckmittel eine von der ersten Gehäusehälfte gebildete erste Streckkante und/oder eine von der zweiten Gehäusehälfte gebildete zweite Streckkante. Die erste Streckkante und/oder die zweite Streckkante definieren einen Spalt, durch den die Materialbahn geführt wird, wenn die Materialbahn aufgewickelt wird. Die erste Streckkante und/oder die zweite Streckkante treten dabei in Kontakt mit der Materialbahn und bringen eine gewisse Reibung auf dieselbe auf, wodurch die Materialbahn vorgestreckt wird. Infolgedessen lässt sich die Materialbahn sehr gleichmäßig Aufwickeln. Die Ausgestaltung der Streckmittel als eine erste Streckkante und/oder als eine zweite Streckkante lässt sich technisch sehr einfach realisieren. Zudem nehmen die Streckkanten wenig Platz in Anspruch.

In einer weitergebildeten Ausführungsform umfassen die Streckmittel
- die erste Gehäusehälfte einen ersten Gleitführungsbereich bildet, der sich der ersten Streckkante zum Hohlraum hin anschließt, und/oder
- die zweite Gehäusehälfte einen zweiten Gleitführungsbereich bildet, der sich der zweiten Streckkante zum Hohlraum hin anschließt.

Der erste Gleitführungsbereich und/oder der zweite Gleitführungsbereich bewirken eine Führung der Materialbahn unter Aufrechterhaltung der Vorspannung, wodurch sich die Materialbahn noch gleichmäßiger aufwickeln lässt.

Bei einer weitergebildeten Ausführungsform
- steht die erste Streckkante über den ersten Gleitführungsbereich über und/oder
- steht die zweite Streckkante über den zweiten Gleitführungsbereich über.

Es hat sich herausgestellt, dass mit der Anordnung der Gleitführungsbereiche in Bezug auf die Streckkanten gemäß dieser Ausführungsform die Materialbahn besonders gleichmäßig aufgewickelt werden kann.

Bei einer weiteren Ausführungsform können der erste Gleitführungsbereich und/oder der zweite Gleitführungsbereich von einer Rippenstruktur oder Wabenstruktur gebildet werden. Die Rippenstruktur oder die Wabenstruktur verleihen der ersten Gehäusehälfte und/oder der zweiten Gehäusehälfte eine zusätzliche Stabilität und insbesondere eine erhöhte Verwindungssteifigkeit, die sich wiederum positiv auf die Gleichmäßigkeit des Aufwickelns der Materialbahn auswirkt.

Eine weitergebildete Ausführungsform zeichnet sich dadurch aus, dass die Vorrichtung eine Seitenführung umfasst, mit welcher die Materialbahn beim Einziehen in den Hohlraum zumindest an einem ihrer Seitenränder geführt wird. Die Seitenführung bewirkt eine Führung der Materialbahn beim Einziehen, so dass selbst dann, wenn die Materialbahn nicht ideal zur Wickeleinheit positioniert wird, die Materialbahn sehr gleichmäßig und ohne seitlichen Versatz aufgewickelt wird.

Nach Maßgabe einer weiteren Ausführungsform umfasst die Seitenführung ein erstes Seitenführungselement und ein zweites Seitenführungselement, wobei zumindest das erste Seitenführungselement zum zweiten Seitenführungselement hin von diesem weg bewegbar im Gehäuse gelagert ist. In dieser Ausführungsform wird die Materialbahn an beiden Seitenrändern geführt, wodurch ein seitlicher Versatz beim Aufwickeln besonders effektiv verhindert wird. Aufgrund der Tatsache, dass das erste Seitenführungselement und das zweite Seitenführungselement relativ zueinander bewegt werden können, können Materialbahnen von unterschiedlicher Breite optimal mit der vorliegenden Vorrichtung aufgewickelt werden. Die Vorrichtung lässt sich daher flexibel einsetzen.

Eine weitere Ausführungsform zeichnet sich dadurch aus, dass
- die erste Gehäusehälfte und die zweite Gehäusehälfte mittels eines Scharniers um eine Gehäusedrehachse drehbar miteinander verbunden sind, wobei
- das Scharnier eine Scharnierwelle umfasst, an welcher
   o die erste Gehäusehälfte und die zweite Gehäusehälfte um die Gehäusedrehachse drehbar, und
   o das erste Seitenführungselement und/oder das zweite Seitenführungselement entlang der Gehäusedrehachse längsverschiebbar gelagert sind.

In dieser Ausführungsform dient die Scharnierwelle einerseits zur drehbaren Lagerung der beiden Gehäusehälften relativ zueinander, andererseits jedoch auch zur längsverschiebbaren Lagerung des ersten Seitenführungselements und/oder des zweiten Seitenführungselements. Aufgrund der Doppelfunktion der Scharnierwelle kann die Anzahl der benötigten Bauteile niedrig gehalten werden, wodurch sich der Herstellungsaufwand ebenfalls niedrig halten lässt.

Nach Maßgabe einer weiteren Ausführungsform weist die Wickeleinheit zwei Wickelstäbe auf, die
- parallel zueinander und parallel zur Gehäusedrehachse verlaufen,
- um die Gehäusedrehachse drehbar im Gehäuse gelagert sind, und
- einen Zwischenraum bilden, wobei
- die Materialbahn zum Aufwickeln in den Zwischenraum einbringbar ist.

Um die Materialbahn mit der Wickeleinheit aufwickeln zu können, muss die Materialbahn mit einem ersten Ende an der Wickeleinheit befestigt werden. Denkbar wäre, hierzu Klemmen an der auf Wickeleinheit vorzusehen, welche aber einerseits vergleichsweise kompliziert in der Herstellung sind und andererseits die Handhabung verkomplizieren. Das Vorsehen von zwei parallel zueinander verlaufenden Wickelstäbe macht hingegen die Befestigung der Materialbahn mit einem Ende an der Wickeleinheit besonders einfach. Hierzu muss die Materialbahn mit einem Ende lediglich ein kleines Stück durch den Zwischenraum zwischen den beiden Wickelstäben hindurchgeführt werden. Beim Aufwickeln befestigt sich die Materialbahn selbst an der Wickeleinheit, ohne dass der Benutzer weitere Maßnahmen ergreifen muss.

In einer weitergebildeten Ausführungsform können die Wickelstäbe mittels eines Wickellagers im Gehäuse gelagert sein, wobei das Gehäuse einen ersten Wickellageraufnahmeabschnitt und einen zweiten Wickellageraufnahmeabschnitt bildet, wobei das Wickellager in den ersten Wickellageraufnahmeabschnitt oder in den zweiten Wickellageraufnahmeabschnitt lösbar einsetzbar ist. Die Möglichkeit, dass Wickellager in den ersten Wickellageraufnahmeabschnitt oder den zweiten Wickellageraufnahmeabschnitt einzusetzen, ermöglicht es dem Benutzer, die Wickeleinheit so anzuordnen, wie es für ihn zum Drehen der Wickelstäbe am angenehmsten ist. Insbesondere lässt sich die Wickeleinheit so ausrichten, dass sie für einen Rechtshänder oder einen Linkshänder besonders gut zu bedienen ist.

Bei einer weitergebildeten Ausführungsform kann die Vorrichtung eine Kurbel umfassen, die zum Drehen der Wickelstäbe mit dem Wickellager zusammenwirkt. Die Verwendung einer Kurbel ermöglicht es auf einfache Weise, die Wickelstäbe mit einem geringen Kraftaufwand zu drehen.

Bei einer weiteren Ausführungsform kann die Kurbel zwischen einer ersten Stellung und einer zweiten Stellung bewegbar am Wickellager befestigt sein. Die erste Stellung kann dabei beispielsweise eine Transportstellung und die zweite Stelle eine Gebrauchsstellung sein. In der Transportstellung kann die Kurbel so angeordnet werden, dass sie kaum über das Gehäuse übersteht, wodurch Platz eingespart und die Wahrscheinlichkeit, dass sie mit anderen Gegenständen kollidiert und dadurch beschädigt wird, verringert wird. In der Gebrauchsstellung hingegen kann die Kurbel so angeordnet werden, dass sie bequem vom Benutzer ergriffen werden kann.

Eine weitergebildete Ausführungsform zeichnet sich dadurch aus, dass
- das Wickellager eine Wickellagergleitfläche aufweist, mit welcher das Wickellager mit dem ersten Wickellageraufnahmeabschnitt und/oder dem zweiten Wickellageraufnahmeabschnitt zusammenwirkt, wobei
- die Wickellagergleitfläche, der erste Wickellageraufnahmeabschnitt und/oder der zweite Wickellageraufnahmeabschnitt eine reibungsreduzierte Oberfläche aufweisen.

Die reibungsreduzierte Oberfläche hat den Effekt, dass sich die Wickeleinheit mit einem besonders geringen Kraftaufwand betätigen lässt. Die reibungsreduzierte Oberfläche kann beispielsweise mittels einer Beschichtung der Wickellagergleitfläche, des ersten Wickellageraufnahmeabschnitts und/oder des zweiten Wickellageraufnahmeabschnitts mit Polytetrafluorethylen (PTFE) bereitgestellt werden. Alternativ kann die reibungsreduzierte Oberfläche dadurch bereitgestellt werden, dass das Spritzgusswerkzeug, mit welchem die Gehäusehälften gefertigt sind, an der betreffenden Gegenfläche mit einer besonders geringen Oberflächenrauheit versehen ist. Beim Spritzgießen überträgt sich die besonders glatte Oberfläche des Spritzgusswerkzeugs auf die betreffende Gehäusehälfte.

Nach Maßgabe einer weiteren Ausführungsform
- bildet die erste Gehäusehälfte eine zum Hohlraum hinweisende erste Vertiefung, und/oder
- bildet die zweite Gehäusehälfte eine zum Hohlraum hinweisende zweite Vertiefung.

Die erste Vertiefung und/oder die zweite Vertiefung wirken als Griffabschnitte, mit welchen der Benutzer die Vorrichtung gut ergreifen kann, wobei die Gefahr, dass die Vorrichtung dem Benutzer aus der Hand rutscht, verringert wird.

Eine weitere Ausführungsform zeichnet sich dadurch aus, dass
- die erste Gehäusehälfte eine erste Außenoberfläche und die zweite Gehäusehälfte eine zweite Außenoberfläche bilden, wobei
- die erste Außenoberfläche und/oder die zweite Außenoberfläche als eine superhydrophobe Oberfläche ausgebildet sind.

Superhydrophobe Oberflächen zeichnen sich dadurch aus, dass sie die Anlagerung von Partikeln, insbesondere von Bakterien und Viren, wirkungsvoll verhindern. Infolgedessen lassen sich die superhydrophoben Oberflächen besonders gut reinigen und frei von Krankheitserregern halten. Die Verbreitung von Krankheitserregern und von den von ihnen hervorgerufenen Krankheiten lässt sich hierdurch deutlich verringern. Die Eigenschaft, dass ein Anhaften von Krankheitserregern und anderen Partikeln an den superhydrophobe Oberflächen verhindert wird, wird häufig auch als Lotus-Effekt bezeichnet.

Dabei bietet es sich an, das Gehäuse aus einem spritzgussfähigen Kunststoff herzustellen. Superhydrophobe Oberflächen können beispielsweise mittels einer entsprechenden Textur im Spritzgusswerkzeug erzeugt werden.

Beispielhafte Ausführungsformen der Erfindung werden im Folgenden unter Bezugnahme auf die beigefügten Zeichnungen näher erläutert. Es zeigen
- Figur 1: eine Explosionsansicht eines ersten Ausführungsbeispiels einer erfindungsgemäßen Vorrichtung zum Handhaben von bandförmigen Materialbahnen, insbesondere von Binden,
- Figur 2: eine Hinteransicht der in Figur 1 gezeigten Vorrichtung,
- Figur 3: eine Schnittdarstellung der Vorrichtung entlang der in Figur 2 definierten Schnittebene A-A,
- Figur 4: eine erste Seitenansicht der in Figur 1 dargestellten Vorrichtung,
- Figur 5: eine zweite Seitenansicht der in Figur 1 dargestellten Vorrichtung,
- Figur 6: eine Schnittdarstellung der Vorrichtung entlang der in Figur 5 definierten Schnittebene B-B,
- Figur 7: eine perspektivische Darstellung der in Figur 1 dargestellten Vorrichtung,
- Figur 8: eine Vorderansicht der in Figur 1 gezeigten Vorrichtung,
- Figur 9: eine Draufsicht der in Figur 1 gezeigten Vorrichtung,
- Figur 10: eine Explosionsansicht eines zweiten Ausführungsbeispiels einer erfindungsgemäßen Vorrichtung zum Handhaben von bandförmigen Materialbahnen,
- Figur 11: eine perspektivische Darstellung des in Figur 10 dargestellten Ausführungsbeispiels im montierten Zustand, und
- Figur 12: eine Seitenansicht des in Figur 11 dargestellte Ausführungsbeispiels in der Schließstellung.

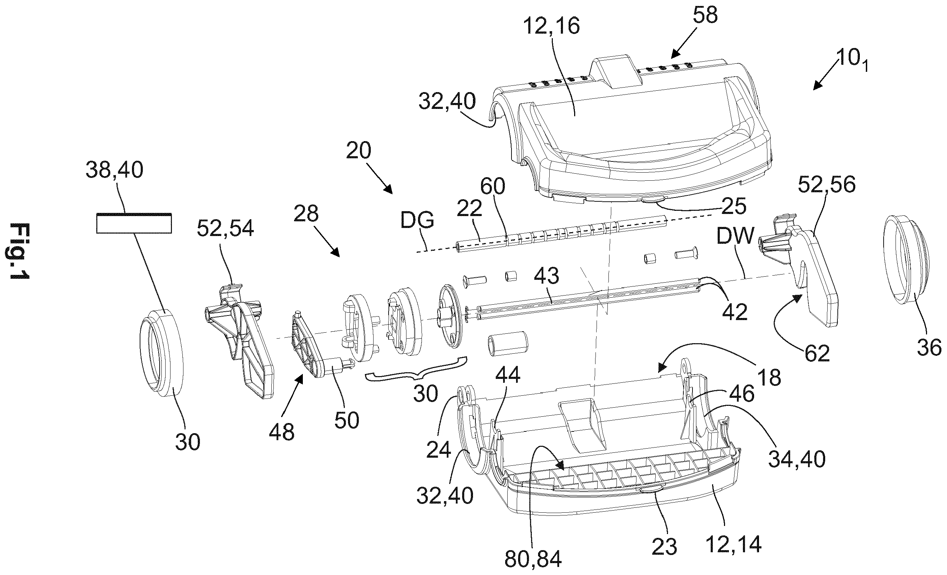

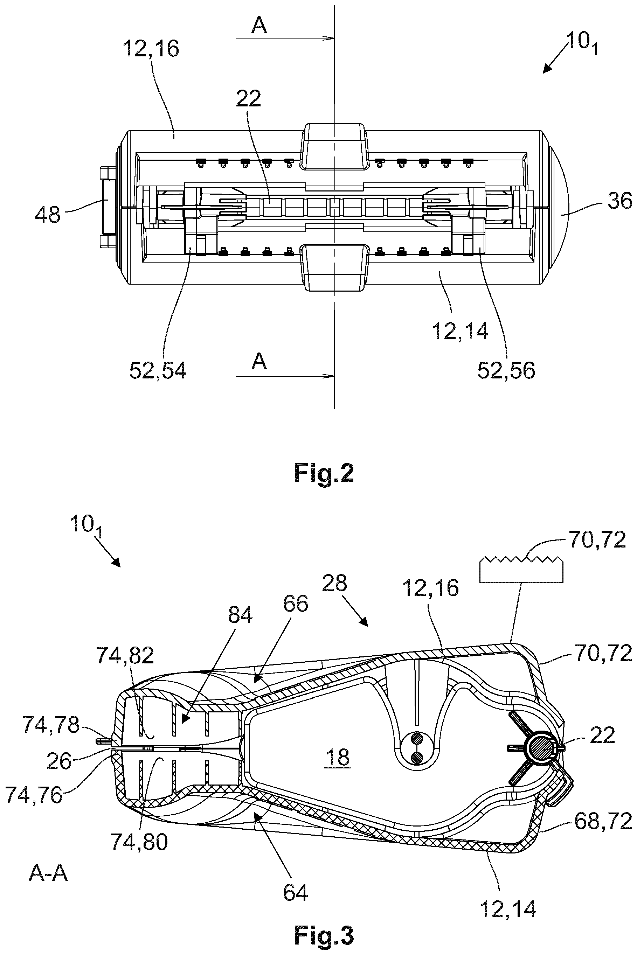

Die Figuren 1 bis 9 zeigen ein erstes Ausführungsbeispiel einer Vorrichtung 10₁ zum Handhaben von bandförmigen Materialbahnen, insbesondere von elastischen Binden. Die Vorrichtung 10₁ umfasst ein Gehäuse 12 mit einer ersten Gehäusehälfte 14 und einer zweiten Gehäusehälfte 16, die einen Hohlraum 18 umschließen, der in der Figur 3 besonders gut zu erkennen ist. Die erste Gehäusehälfte 14 und die zweite Gehäusehälfte 16 sind im dargestellten ersten Ausführungsbeispiel identisch.

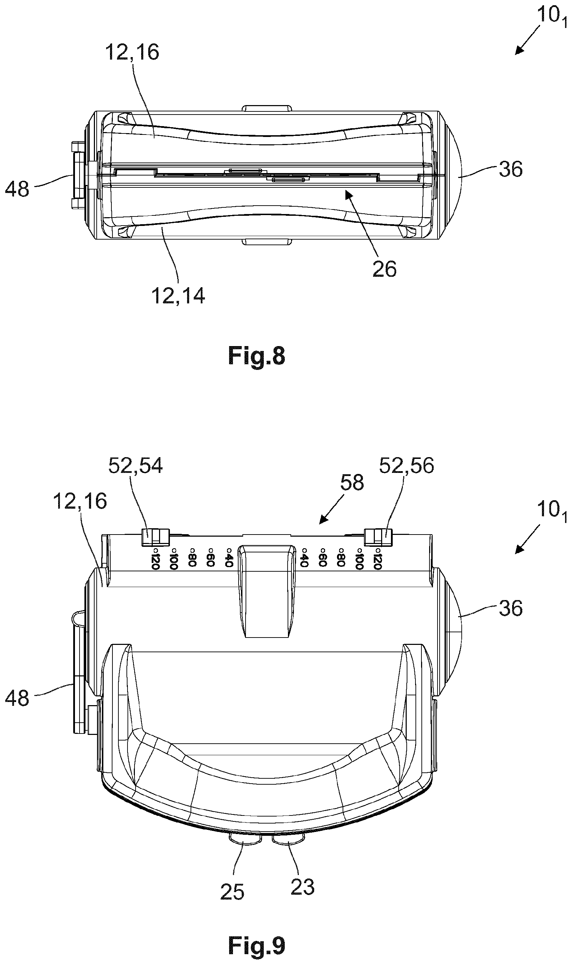

Die erste Gehäusehälfte 14 und die zweite Gehäusehälfte 16 sind mittels eines Scharniers 20 zwischen einer Offenstellung und einer Schließstellung um eine Gehäusedrehachse DG drehbar miteinander verbunden. In den Figuren 2 bis 9 ist die Vorrichtung 10₁ in der Schließstellung gezeigt, wobei die Explosionsdarstellung der Figur 1 ebenfalls an die Schließstellung anknüpft. Die Gehäusedrehachse DG wird von einer Scharnierwelle 22 definiert, welche in Scharnierösen 24 der ersten Gehäusehälfte 14 und der zweiten Gehäusehälfte 16 eingebracht werden kann. Zum vereinfachten Überführen von der Schließstellung in die Offenstellung weist die erste Gehäusehälfte 14 einen ersten Vorsprung 23 und die zweite Gehäusehälfte 16 einen zweiten Vorsprung 25 auf (siehe Figuren 1 und 9).

Wie beispielsweise aus der Figur 3 zu erkennen ist, wird in der Schließstellung ein spaltförmiger Durchtrittsabschnitt 26 vom Gehäuse 12 gebildet, durch welchen der Hohlraum 18 zugänglich ist. Im Hohlraum 18 ist eine Wickeleinheit 28 angeordnet, welche mittels eines Wickellagers 30 drehbar im Gehäuse 12 gelagert ist. Mit der Wickeleinheit 28 kann eine hier nicht dargestellte Materialbahnen, insbesondere eine elastische Binde, in den Hohlraum 18 eingezogen und aufgewickelt werden. Hierauf wird später noch genauer eingegangen.

Zum Lagern der Wickeleinheit 28 ist das Wickellager 30 in einen ersten Wickellageraufnahmeabschnitt 32 oder in einen zweiten Wickellageraufnahmeabschnitt 34 einsetzbar, die vom Gehäuse 12 gebildet werden. Hierzu werden die erste Gehäusehälfte 14 und die zweite Gehäusehälfte 16 in die Offenstellung überführt. Im in den Figuren 1 bis 9 dargestellten ersten Ausführungsbeispiel erstreckten sich die beiden Wickellageraufnahmeabschnitte 32, 34 auf die erste Gehäusehälfte 14 und die zweite Gehäusehälfte 16, wobei die erste Gehäusehälfte 14 und die zweite Gehäusehälfte 16 pro Wickellageraufnahmeabschnitt 32, 34 jeweils eine halbkreisförmige Ausnehmung bilden. Das Wickellager 30 kann wahlweise in den ersten Wickellageraufnahmeabschnitt 32 oder in den zweiten Wickellageraufnahmeabschnitt 34 eingesetzt werden. Ein Deckel 36 kann in denjenigen Wickellageraufnahmeabschnitt 32, 34 eingesetzt werden, der nicht für das Wickellager 30 verwendet wird.

Das Wickellager 30 bildet eine Wickellagergleitfläche 38, mit welcher das Wickellager 30 mit dem ersten Wickellageraufnahmeabschnitt 32 und dem zweiten Wickellageraufnahmeabschnitt 34 in Kontakt tritt, wenn das Wickellager 30 in einen dieser Wickellageraufnahmeabschnitte 32, 34 eingesetzt ist. Die Wickellagergleitfläche 38, der erste Wickellageraufnahmeabschnitt 32 und/oder der zweite Wickellageraufnahmeabschnitt 34 weisen eine reibungsreduzierte Oberfläche 40 auf, welche in der Figur 1 prinzipiell dargestellt ist. Die reibungsreduzierte Oberfläche 40 kann beispielsweise als eine PTFE-Beschichtung ausgebildet sein.

Am Wickellager 30 sind zwei Wickelstäbe 42 drehfest befestigt, welche zusammen mit dem Wickellager 30 um eine Wickeldrehachse DW drehbar sind, wobei die Wickeldrehachse DW parallel zur Gehäusedrehachse DG verläuft. Die beiden Wickelstäbe 42 bilden einen Zwischenraum 43, auf dessen Funktion später noch genauer eingegangen wird. Das Gehäuse 12 bildet eine erste Abstützung 44 und eine zweite Abstützung 46, auf welchen sich die Wickelstäbe 42 abstützen können (siehe Figuren 1 und 3). Um eine möglichst reibungsarme und gut definierte Führung der Wickelstäbe 42 in den beiden Abstützung 44, 46 zu erreichen, können entsprechend ausgebildete Lagerbuchsen (nicht dargestellt) vorgesehen sein, durch welche die beiden Wickelstäbe 42 weitgehend spielfrei durchgeführt werden können.

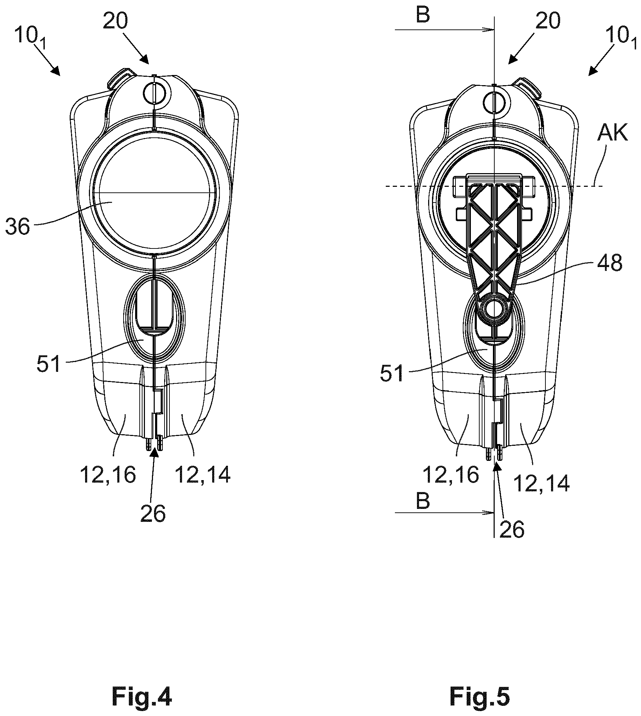

Darüber hinaus ist am Wickellager 30 eine Kurbel 48 mit einem Kurbelgriff 50 befestigt, welche zwischen einer ersten Stellung und einer zweiten Stellung bewegbar ist. In sämtlichen Figuren befindet sich die Kurbel 48 in einer ersten Stellung, welche im in den Figuren 1 bis 9 dargestellten ersten Ausführungsbeispiel eine Transportstellung darstellt. In der Transportstellung ragt der Kurbelgriff 50 in einen Gehäusedurchbruch 51 hinein, so dass der Kurbelgriff 50 nicht von der Kurbel 48 absteht und an Gegenständen in der Umgebung der Vorrichtung 10₁ hängen bleiben könnte. Die zweite Stellung, welche in den Figuren nicht dargestellt ist, ist die Gebrauchsstellung, in welcher ein Benutzer das Wickellager 30 mittels der Kurbel 48 drehen kann. Um die Kurbel 48 von der Transportstellung in die Gebrauchsstellung zu bewegen, wird diese in etwa um 180° um eine Kurbelachse AK (siehe Figur 5)gedreht, wobei die Kurbelachse senkrecht zur Wickeldrehachse DW verläuft

Zudem ist die Vorrichtung 10₁ mit einer Seitenführung 52 ausgestattet, welche insbesondere aus der Figur 1 gut zu erkennen ist. Die Seitenführung 52 umfasst ein erstes Seitenführungselement 54 und ein zweites Seitenführungselement 56, welche an der Scharnierwelle 22 befestigt und entlang der Scharnierwelle 22 und somit entlang der Gehäusedrehachse DG bewegbar sind. Insbesondere aus der Figur 9 geht hervor, dass das Gehäuse 12 mit einer Skala 58 versehen ist, mit welcher die Position der Seitenführungselemente 54, 56 relativ zueinander bestimmt werden kann. Um typische Positionen der Seitenführungselemente 54, 56 vorgeben zu können, weist die Scharnierwelle 22 Nuten 60 auf, in welche die Seitenführungselemente 54, 56 einrasten können. In den typischen Positionen weisen die Seitenführungselemente 54, 56 Abstände zueinander auf, welche den Breiten von gängigen Binden entsprechen. Darüber hinaus weisen das erste Seitenführungselement 54 und das zweite Seitenführungselement 56 Ausnehmungen 62 auf, welche von den Wickelstäben 42 durchlaufen werden.

Beispielsweise aus der Figur 3 geht hervor, dass die erste Gehäusehälfte 14 eine erste Vertiefung 64 und die zweite Gehäusehälfte 16 eine zweite Vertiefung 66 bilden, welche zum Hohlraum 18 hin ausgerichtet sind. Die erste Vertiefung 64 und die zweite Vertiefung 66 formen einen Griffabschnitt, mit welchem ein Benutzer der Vorrichtung 10₁ dieselbe gut ergreifen kann.

Die erste Gehäusehälfte 14 bildet eine erste Außenoberfläche 68 und die zweite Gehäusehälfte 16 eine zweite Außenoberfläche 70, wobei die erste Außenoberfläche 68 und die zweite Außenoberfläche 70 vom Hohlraum 18 weg zeigen und diejenigen Flächen sind, welche ein Benutzer der Vorrichtung 10₁ hauptsächlich berührt. Sowohl die erste Außenoberfläche 68 als auch die zweite Außenoberfläche 70 sind jeweils als eine superhydrophobe Oberfläche 72 ausgebildet, welche in Figur 3 prinzipiell dargestellt ist.

Wie eingangs erwähnt, bilden die erste Gehäusehälfte 14 und die zweite Gehäusehälfte 16 in der Schließstellung den spaltförmigen Durchtrittsabschnitt 26, welcher insbesondere aus der Figur 3 gut zu erkennen ist. Wie ebenfalls erwähnt, kann mittels der Wickeleinheit 28 eine Materialbahn durch den Durchtrittsabschnitt 26 in den Hohlraum 18 eingezogen und aufgewickelt werden. Hierzu kann beispielsweise auf folgende Weise vorgegangen werden:
Zunächst werden die beiden Gehäusehälften in die Offenstellung bewegt. Anschließend wird ein Ende der Materialbahn in den Zwischenraum 43 zwischen die beiden Wickelstäbe 42 eingebracht und das Wickellager 30 in eine der beiden Wickellageraufnahmeabschnitte 32, 34 eingesetzt. Welcher Wickellageraufnahmeabschnitt 32, 34 verwendet wird, hängt beispielsweise davon ab, ob der Benutzer Rechtshänder oder Linkshänder ist. In denjenigen Wickellageraufnahmeabschnitt 32, 34, der dann frei bleibt, wird der Deckel 36 eingesetzt. Anschließend werden die beiden Gehäusehälften 14, 16 in die Schließstellung überführt. Infolgedessen sind das Wickellager 30 und der Deckel 36 vollumfänglich von der ersten Gehäusehälfte 14 und der zweiten Gehäusehälfte 16 umschlossen.

Das erste Seitenführungselement 54 und das zweite Seitenführungselement 56 werden nun so entlang der Scharnierwelle 22 verschoben, dass die seitlichen Ränder der Materialbahn am ersten Seitenführungselement 54 und am zweiten Seitenführungselement 56 anliegen, ohne die Materialbahn zu stauchen.

Die Kurbel 48 wird in die Gebrauchsstellung gestellt, so dass nun durch eine entsprechende Drehbewegung die Wickeleinheit 28 und folglich die beiden Wickelstäbe 42 um die Wickeldrehachse DW gedreht werden können. Die Materialbahn wird nun um die Wickelstäbe 42 gewickelt, wobei die Materialbahn durch den Durchtrittsabschnitt 26 in den Hohlraum 18 eingezogen und dabei vom ersten Seitenführungselement 54 und vom zweiten Seitenführungselement 56 geführt wird.

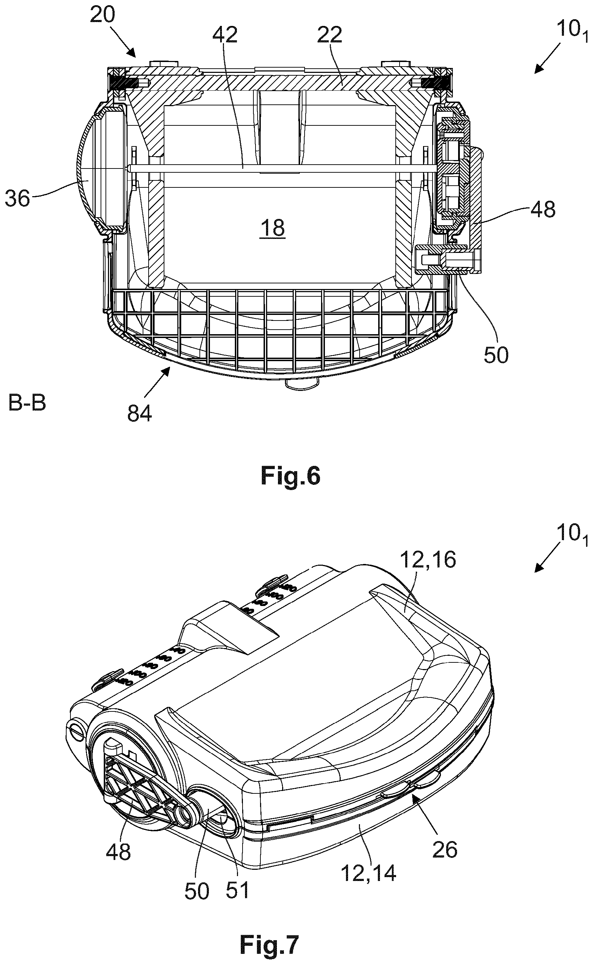

Im Durchtrittsabschnitt 26 ist ein Streckmittel 74 angeordnet, welches beim Einziehen derart mit der Materialbahn zusammenwirkt, dass eine Materialspannung auf die Materialbahn aufgebracht wird. Wie insbesondere aus der Figur 3 zu erkennen ist, umfasst das Streckmittel 74 hierzu eine von der ersten Gehäusehälfte 14 gebildete erste Streckkante 76 und eine von der zweiten Gehäusehälfte 16 gebildete zweite Streckkante 78. Wie ebenfalls aus Figur 3 zu erkennen ist, schließt sich ein erster Gleitführungsbereich 80 der ersten Streckkante 76 zum Hohlraum 18 hin an. Entsprechend schließt sich zum Hohlraum 18 hin ein zweiter Gleitführungsbereich 82 der zweiten Streckkante 78 an. Dabei steht die erste Streckkante 76 über den ersten Gleitführungsbereich 80 über. Entsprechend steht die zweite Streckkante 78 über den zweiten Gleitführungsbereich 82 über. Aus den Figuren 1 und 6 ist erkennbar, dass der erste Gleitführungsbereich 80 und der zweite Gleitführungsbereich 82 von einer Rippenstruktur 84 gebildet werden.

Die erste Streckkante 76 und/oder die zweite Streckkante 78 kommen mit der Materialbahn beim Einziehen in den Hohlraum 18 in Kontakt und erzeugen somit eine gewisse Reibung, wodurch die Materialbahn vorgespannt wird. Die Rippenstruktur 84 sorgt dabei für eine besonders hohe Verwindungssteifigkeit der ersten Gehäusehälfte 14 und der zweiten Gehäusehälfte 16. Die Ausgestaltung der Streckmittel 74 trägt dazu bei, dass die Materialbahn mit einer gewissen Vorspannung gleichmäßig aufgewickelt wird.

Die Wickeleinheit 28 wird solange gedreht, bis dass die Materialbahn vollständig in den Hohlraum 18 eingezogen und auf die Wickelstäbe 42 aufgewickelt ist. Dann wird das Gehäuse 12 geöffnet, wozu die erste Gehäusehälfte 14 und die zweite Gehäusehälfte 16 in die Offenstellung gebracht werden. Das Wickellager 30 wird nun aus dem ersten Wickellageraufnahmeabschnitt 32 oder dem zweiten Wickellageraufnahmeabschnitt 34 entnommen. Infolgedessen wird auch die aufgewickelte Materialbahn aus dem Gehäuse 12 entfernt. Die aufgewickelte Materialbahn kann nun über das freie Ende der beiden Wickelstäbe 42 von der Wickeleinheit 28 getrennt und bis zur nächsten Verwendung gelagert werden. Anschließend kann entweder eine weitere Materialbahn auf die oben beschriebene Weise aufgewickelt werden oder, wenn keine weitere Materialbahn aufgewickelt werden soll, das Wickellager 30 wieder in den betreffenden Wickellageraufnahmeabschnitt eingesetzt und die erste Gehäusehälfte 14 und die zweite Gehäusehälfte 16 in die Schließstellung überführt werden. Zudem kann in diesem Fall die Kurbel 48 wieder in die Transportstellung gedreht und die Vorrichtung 10₁ gelagert oder zum nächsten Einsatzort transportiert werden.

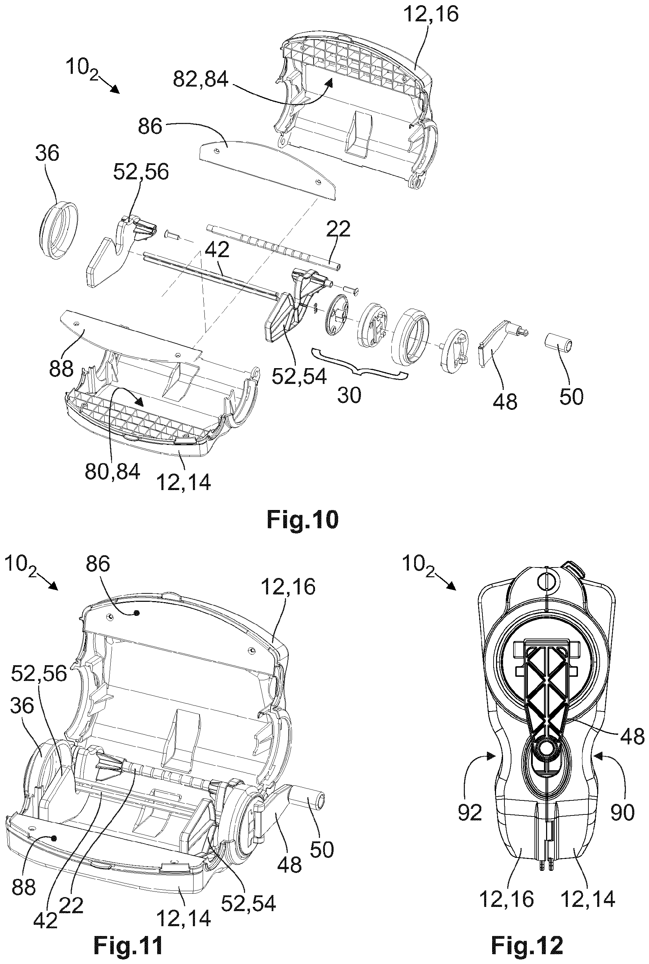

In den Figuren 10 bis 12 ist ein zweites Ausführungsbeispiel der erfindungsgemäßen Vorrichtung 10₂ in verschiedenen Ansichten gezeigt. Der Wesentliche Aufbau und die Funktionsweise der Vorrichtung 10₂ nach dem zweiten Ausführungsbeispiel entsprechen dabei weitgehend denjenigen des ersten Ausführungsbeispiels, so dass im Folgenden nur auf die wesentlichen Unterschiede eingegangen wird.

In Figur 10 ist die Vorrichtung 10₂ nach dem zweiten Ausführungsbeispiel in einer Explosionsdarstellung gezeigt, wobei sich die Explosionsdarstellung an der Offenstellung der Vorrichtung 10₂ anlehnt. Man erkennt, dass auch die Vorrichtung 10₂ nach dem zweiten Ausführungsbeispiel die bereits erwähnte Rippenstruktur 84 oder Wabenstruktur aufweist, welche den ersten Gleitführungsbereich 80 und den zweiten Gleitführungsbereich 82 bereitstellt. In Figur 10 ist zu erkennen, dass die Vorrichtung 10₂ eine erste Gleitführungsfläche 86 und eine zweite Gleitführungsfläche 88 umfasst, die nach Art einer Abdeckung ausgestaltet sind. Aus Figur 11, welche die Vorrichtung 10₂ in der Offenstellung zeigt, ist erkennbar, dass die erste Gleitführungsfläche 86 und die zweite Gleitführungsfläche 88 auf die Rippenstruktur 84 aufgesetzt und dort mit der ersten Gehäusehälfte 14 bzw. mit der zweiten Gehäusehälfte 16 verbunden werden. Die Rippenstruktur 84 wird infolgedessen verdeckt. Die beim Aufwickeln der Binde auf diese wirkende Oberfläche wird hierdurch vergrößert, so dass sich der führende Effekt der Gleitführungsbereiche 80, 82 erhöhen und die Vorspannung beibehalten lässt. Zudem wird die Ansammlung von Schmutz in der Rippenstruktur 84 verhindert.

In Figur 12 ist eine Seitenansicht der Vorrichtung 10₂ nach dem zweiten Ausführungsbeispiel in der Schließstellung gezeigt. Insbesondere im Vergleich zu den Figuren 4 und 5, welche die Vorrichtung 10₁ nach dem ersten Ausführungsbeispiel aus vergleichbaren Perspektiven zeigen, ist erkennbar, dass die erste Gehäusehälfte 14 eine erste Mulde 90 und die zweite Gehäusehälfte 16 eine zweite Mulde 92 aufweisen. Der Benutzer, der die Vorrichtung 10₂ nach dem zweiten Ausführungsbeispiel zum Aufwickeln in der Schließstellung ergreift, kann seinen Daumen in die erste Mulde 90 und seinen Zeigefinger in die zweite Mulde 92 oder umgekehrt legen, wodurch der Halt im Vergleich zur Vorrichtung 10₁ nach dem ersten Ausführungsbeispiel verbessert und verhindert wird, dass dem Benutzer die Vorrichtung 10₂ beim Aufwickeln aus der Hand rutscht.

### Bezugszeichenliste

- 10: Vorrichtung
- 10₁, 10₂: Vorrichtung
- 12: Gehäuse
- 14: erste Gehäusehälfte
- 16: zweite Gehäusehälfte
- 18: Hohlraum

- 20: Scharnier
- 22: Scharnierwelle
- 23: erster Vorsprung
- 24: Scharnieröse
- 25: zweiter Vorsprung
- 26: Durchtrittsabschnitt
- 28: Wickeleinheit

- 30: Wickellager
- 32: erster Wickellageraufnahmeabschnitt
- 34: zweiter Wickellageraufnahmeabschnitt
- 36: Deckel
- 38: Wickellagergleitfläche

- 40: reibungsreduzierte Oberfläche
- 42: Wickelstab
- 43: Zwischenraum
- 44: erste Abstützung
- 46: zweite Abstützung
- 48: Kurbel

- 50: Kurbelgriff
- 51: Gehäusedurchbruch
- 52: Seitenführung
- 54: erstes Seitenführungselement
- 56: zweites Seitenführungselement
- 58: Skala
- 60: Nut
- 62: Ausnehmung
- 64: erste Vertiefung
- 66: zweite Vertiefung
- 68: erste Außenoberfläche

- 70: zweite Außenoberfläche
- 72: superhydrophobe Oberfläche
- 74: Streckmittel
- 76: erste Streckkante
- 78: zweite Streckkante

- 80: erster Gleitführungsbereich
- 82: zweiter Gleitführungsbereich
- 84: Rippenstruktur
- 86: erste Gleitführungsfläche
- 88: zweite Gleitführungsfläche
- 90: erste Mulde
- 92: zweite Mulde

- DG: Gehäusedrehachse
- DW: Wickeldrehachse
- AK: Kurbelachse

## Patentansprüche

1. Vorrichtung (10) zum Handhaben von bandförmigen Materialbahnen, insbesondere von Binden, umfassend
- ein Gehäuse (12) mit einer ersten Gehäusehälfte (14) und einer zweiten Gehäusehälfte (16), die einen Hohlraum (18) umschließen und zwischen einer Offenstellung und einer Schließstellung drehbar miteinander verbindbar oder verbunden sind, wobei in der Schließstellung das Gehäuse (12) einen spaltförmigen Durchtrittsabschnitt (26) bildet, durch welchen der Hohlraum (18) zugänglich ist, und
- eine im Gehäuse (12) drehbar gelagerte Wickeleinheit (28), mit welcher eine Materialbahn durch den Durchtrittsabschnitt (26) in den Hohlraum (18) einziehbar ist und aufwickelbar ist,
**dadurch gekennzeichnet, dass** im Durchtrittsabschnitt (26) mit der Materialbahn beim Einziehen zusammenwirkende Streckmittel (74) zum Aufbringen einer Materialspannung angeordnet sind.

2. Vorrichtung (10) nach Anspruch 1,
**dadurch gekennzeichnet, dass** die Streckmittel (74) eine von der ersten Gehäusehälfte (14) gebildete erste Streckkante (76) und/oder eine von der zweiten Gehäusehälfte (16) gebildete zweite Streckkante (78) umfassen.

3. Vorrichtung (10) nach Anspruch 2,
**dadurch gekennzeichnet, dass**
- die erste Gehäusehälfte (14) einen ersten Gleitführungsbereich (80) bildet, der sich der ersten Streckkante (76) zum Hohlraum (18) hin anschließt, und/oder
- die zweite Gehäusehälfte (16) einen zweiten Gleitführungsbereich (82) bildet, der sich der zweiten Strecckante (78) zum Hohlraum (18) hin anschließt.

4. Vorrichtung (10) nach Anspruch 3,
**dadurch gekennzeichnet, dass**
- die erste Streckkante (76) über den ersten Gleitführungsbereich (80) übersteht und/oder
- die zweite Streckkante (78) über den zweiten Gleitführungsbereich (82) übersteht.

5. Vorrichtung (10) nach einem der Ansprüche 3 oder 4,
**dadurch gekennzeichnet, dass** der erste Gleitführungsbereich (80) und/oder der zweiten Gleitführungsbereich (82) von einer Rippenstruktur (84) oder Wabenstruktur gebildet werden.

6. Vorrichtung (10) nach einem der vorherigen Ansprüche,
**dadurch gekennzeichnet, dass** die Vorrichtung (10) eine Seitenführung (52) umfasst, mit welcher die Materialbahn beim Einziehen in den Hohlraum (18) zumindest an einem ihrer Seitenränder geführt wird.

7. Vorrichtung (10) nach Anspruch 6,
**dadurch gekennzeichnet, dass** die Seitenführung (52) ein erstes Seitenführungselement (54) und ein zweites Seitenführungselement (56) umfasst, wobei zumindest das erste Seitenführungselement (54) zum zweiten Seitenführungselement (56) hin von diesem weg bewegbar im Gehäuse (12) gelagert ist.

8. Vorrichtung (10) nach Anspruch 7,
**dadurch gekennzeichnet, dass**
- die erste Gehäusehälfte (14) und die zweite Gehäusehälfte (16) mittels eines Scharniers (20) um eine Gehäusedrehachse (DG) drehbar miteinander verbunden sind, wobei
- das Scharnier (20) eine Scharnierwelle umfasst, an welcher
∘ die erste Gehäusehälfte (14) und die zweite Gehäusehälfte (16) um die Gehäusedrehachse (DG) drehbar, und
∘ das erste Seitenführungselement (54) und/oder das zweite Seitenführungselement (56) entlang der Gehäusedrehachse (DG) längsverschiebbar gelagert sind.

9. Vorrichtung (10) nach Anspruch 8,
**dadurch gekennzeichnet, dass** die Wickeleinheit (28) zwei Wickelstäbe (42) aufweist, die
- parallel zueinander und parallel zur Gehäusedrehachse (DG) verlaufen,
- um eine parallel zur Gehäusedrehachse (DG) verlaufende Wickeldrehachse (DW) drehbar im Gehäuse (12) gelagert sind, und
- einen Zwischenraum (43) bilden, wobei
- die Materialbahn zum Aufwickeln in den Zwischenraum (43) einbringbar ist.

10. Vorrichtung (10) nach Anspruch 9,
**dadurch gekennzeichnet, dass** die Wickelstäbe (42) mittels eines Wickellagers (30) im Gehäuse (12) gelagert sind, wobei das Gehäuse (12) einen ersten Wickellageraufnahmeabschnitt (32) und einen zweiten Wickellageraufnahmeabschnitt (34) bildet, wobei das Wickellager (30) in den ersten Wickellageraufnahmeabschnitt (32) oder in den zweiten Wickellageraufnahmeabschnitt (34) lösbar einsetzbar ist.

11. Vorrichtung (10) nach Anspruch 10,
**dadurch gekennzeichnet, dass** die Vorrichtung (10) eine Kurbel (48) umfasst, die zum Drehen der Wickelstäbe (42) mit dem Wickellager (30) zusammenwirkt.

12. Vorrichtung (10) nach Anspruch 11,
**dadurch gekennzeichnet, dass** die Kurbel (48) zwischen einer ersten Stellung und einer zweiten Stellung bewegbar am Wickellager (30) befestigt ist.

13. Vorrichtung (10) nach einem der Ansprüche 10 bis 12,
**dadurch gekennzeichnet, dass**
- das Wickellager (30) eine Wickellagergleitfläche (38) aufweist, mit welcher das Wickellager (30) mit dem ersten Wickellageraufnahmeabschnitt (32) und/oder dem zweiten Wickellageraufnahmeabschnitt (34) zusammenwirkt, wobei
- die Wickellagergleitfläche (38), der erste Wickellageraufnahmeabschnitt (32) und/oder der zweite Wickellageraufnahmeabschnitt (34) eine reibungsreduzierte Oberfläche (40) aufweisen.

14. Vorrichtung (10) nach einem der Ansprüche 10 bis 13,
**dadurch gekennzeichnet, dass**
- die erste Gehäusehälfte (14) eine zum Hohlraum (18) hinweisende erste Vertiefung (64), und/oder
- die zweite Gehäusehälfte (16) eine zum Hohlraum (18) hinweisende zweite Vertiefung (66) bildet.

15. Vorrichtung (10) nach einem der Ansprüche 10 bis 14,
**dadurch gekennzeichnet, dass**
- die erste Gehäusehälfte (14) eine erste Außenoberfläche (68) und die zweite Gehäusehälfte (16) eine zweite Außenoberfläche (70) bilden, wobei
- die erste Außenoberfläche (68) und/oder die zweite Außenoberfläche (70) als eine superhydrophobe Oberfläche (72) ausgebildet sind.

## Claims

1. Apparatus (10) for manipulating band-shaped strips of material, in particular bandages, comprising
- a housing (12), having a first housing half (14) and a second housing half (16), which encompass a hollow space (18), and which are rotatably connectable or connected to each other between an open position and a closed position, wherein, in the closed position, the housing (12) forms a slit-shaped passageway section (26), via which the hollow space (18) is accessible, and
- a winding unit (28), rotatably mounted in the housing (12), with which a strip of material can be drawn through the passageway section (26) into the hollow space (18) and can be wound up,
**characterized in that**, in the passageway section (26), stretching means (74) are arranged which interact with the strip of material when it is drawn in for the application of a material tension.

2. Apparatus (10) in accordance with claim 1,
**characterized in that** the stretching means (74) comprise a first stretching edge (76), formed by the first housing half (14) and/or a second stretching edge (78), formed by the second stretching edge (78).

3. Apparatus (10) in accordance with claim 2,
**characterized in that**
- the first housing half (14) forms a first sliding guide section (80), which follows the first stretching edge (76) in the direction of the hollow space (18), and/or
- the second housing half (16) forms a second sliding guide section (82), which follows the second stretching edge (78) in the direction of the hollow space (18).

4. Apparatus (10) in accordance with claim 3,
**characterized in that**
- the first stretching edge (76) projects over the first sliding guide section (80) and/or
- the second stretching edge (78) projects over the second sliding guide section (82).

5. Apparatus (10) in accordance with either of claims 3 or 4,
**characterized in that** the first sliding guide section (80) and/or the second sliding guide section (82) is formed by a rib structure (84) or by a honeycomb structure.

6. Apparatus (10) in accordance with any of the preceding claims,
**characterized in that** the apparatus (10) comprises a lateral guide (52), by means of which the strip of material is guided along at least one of its side edges when being drawn into the hollow space (18).

7. Apparatus (10) in accordance with claim 6,
**characterized in that** the lateral guide (52) comprises a first lateral guide element (54) and a second lateral guide element (56), wherein at least the first lateral guide element (54) is mounted in the housing (12), in relation to the second lateral guide element (56), moveably away from the latter.

8. Apparatus (10) in accordance with claim 7,
**characterized in that**
- the first housing half (14) and the second housing half (16) are rotatably connected to each other around a housing axis of rotation (DG) by means of a hinge (20), wherein
- the hinge (20) comprises a hinge shaft, on which
o the first housing half (14) and the second housing half (16) are rotatably mounted around the housing axis of rotation (DG), and
o the first lateral guide element (54) and/or the second lateral guide element (56) are longitudinally moveably mounted along the housing axis of rotation (DG).

9. Apparatus (10) in accordance with claim 8,
**characterized in that** the winding unit (28) comprises two winding rods (42), which
- extend parallel to each other and parallel to the housing axis of rotation (DG),
- are rotatably mounted in the housing (12) around a winding axis of rotation (DW) extending parallel to the housing axis of rotation (DG), and
- form an interspace (43), wherein
- the strip of material can be introduced into the interspace (43) in order to be wound up.

10. Apparatus (10) in accordance with claim 9,
**characterized in that** the winding rods (42) are mounted in the housing (12) by means of a winding bearing (30), wherein the housing (12) forms a first winding bearing retainer section (32) and a second winding bearing retainer section (34), wherein the winding bearing (30) in the first winding bearing retainer section (32) or in the second winding bearing retainer section (34) is detachably insertable.

11. Apparatus (10) in accordance with claim 10,
**characterized in that** the apparatus (10) comprises a crank (48) which interacts with the winding bearing (30) in order to rotate the winding rods (42).

12. Apparatus (10) in accordance with claim 11,
**characterized in that** the crank (48) is moveably attached to the winding bearing (30) between a first position and a second position.

13. Apparatus (10) in accordance with any of claim 10 to 12,
**characterized in that**
- the winding bearing (30) comprises a winding bearing slide surface (38), by means of which the winding bearing (30) interacts with the first winding bearing retainer section (32) and/or with the second winding bearing retainer section (34), wherein
- the winding bearing slide surface (38) of the first winding bearing retainer section (32) and/or of the second winding bearing retainer section (34) comprises a friction-reduced surface (40).

14. Apparatus (10) in accordance with any of claim 10 to 13,
**characterized in that**
- the first housing half (14) form a first recess (64) facing the hollow space (18), and/or
- the second housing half (16) forms a second recess (66) facing the hollow space (18).

15. Apparatus (10) in accordance with any of claims 10 to 14,
**characterized in that**
- the first housing half (14) forms a first outer surface (68) and the second housing half (16) forms a second outer surface (70), wherein
- the first outer surface (68) and/or the second outer surface (70) are(is) implemented as a superhydrophobic surface (72).

## Revendications

1. Dispositif (10) pour manutentionner des bandes de matière en forme de rubans, notamment des bandes comprenant :
- un boîtier (12) avec une première moitié de boîtier (14) et une seconde moitié de boîtier (16) entourant une cavité (18) et reliées ou pouvant être reliées entre elles en pivotement entre une position d'ouverture et une position de fermeture,
en position de fermeture, le boîtier (12) formant un segment de passage (26) en forme de fente, à travers lequel on accède à la cavité (18), et
- une unité d'enroulement (28) montée à rotation dans le boîtier (12) pour tirer et enrouler la bande de matière à travers le segment de passage (26) dans la cavité (18),
dispositif **caractérisé par**
des moyens d'étirage (74) coopérant avec la bande de matière dans le segment de passage (26) pour appliquer une tension à la matière lorsqu'elle est tirée.

2. Dispositif (10) selon la revendication 1,
**caractérisé en ce que**
les moyens d'étirage (74) comprennent une première arête d'étirage (76) formée par la première moitié de boîtier (14) et/ou une seconde arête d'étirage (78) formée par la seconde moitié de boîtier (16).

3. Dispositif (10) selon la revendication 2,
**caractérisé en ce que**
- la première moitié de boîtier (14) forme une première zone de guidage glissant (80) qui rejoint la première arête d'étirage (76) vers la cavité (18), et/ou
- la seconde moitié (16) forme une seconde zone de guidage glissant (82) qui fait suite à la première arête d'étirage (78) vers la cavité (18).

4. Dispositif (10) selon la revendication 3,
**caractérisé en ce que**
- la première arête d'étirage (76) dépasse de la première zone de guidage glissant (80), et/ou
- la seconde arête d'étirage (78) dépasse de la seconde zone de guidage glissant (82).

5. Dispositif (10) selon l'une des revendications 3 ou 4,
**caractérisé en ce que**
la première zone de guidage glissant (80) et/ou la seconde zone de guidage glissant (82) sont formées avec une structure de nervure (84) ou une structure en nids d'abeille.

6. Dispositif (10) selon l'une des revendications précédentes,
**caractérisé en ce que**
le dispositif (10) comprend un guide latéral (52) qui guide la bande de matière lors de sa rentrée dans la cavité (18) au moins par l'un de ses bords.

7. Dispositif (10) selon la revendication 6,
**caractérisé en ce que**
le guide latéral (52) comprend un premier élément de guidage latéral (54) et un second élément de guidage latéral (56), au moins le premier élément de guidage latéral (54) étant monté de façon à pouvoir s'écarter du second élément de guidage latéral (56) dans le boîtier (12).

8. Dispositif (10) selon la revendication 7,
**caractérisé en ce que**
la première moitié de boîtier (14) et la seconde moitié de boîtier (16) sont reliées l'une à l'autre par une charnière (20) de pivotement autour d'un axe de rotation de boîtier (DG),
- la charnière (20) comprend un arbre de charnière qui,
- reçoit à rotation la première moitié de boîtier (14) et la seconde moitié de boîtier (16) autour de l'axe de rotation de boîtier (DG), et
* le premier élément de guidage latéral (54) et/ou le second élément de guidage latéral (56) sont montés de façon à coulisser longitudinalement le long de l'axe de rotation de boîtier (DG).

9. Dispositif (10) selon la revendication 8,
**caractérisé en ce que**
l'unité d'enroulement (28) comporte deux tiges d'enroulement (42),
- parallèles l'une à l'autre et parallèles à l'axe de rotation de boîtier (DG),
- montées à rotation dans le boîtier (12) autour d'un axe de rotation d'enroulement (DW) parallèle à l'axe de rotation de boîtier (DG), et
- formant un intervalle (43),
- la bande de matière passant dans l'intervalle (43) pour l'enroulement.

10. Dispositif (10) selon la revendication 9,
**caractérisé en ce que**
les tiges d'enroulement (42) sont montées sur le boîtier (12) dans un palier d'enroulement (30), le boîtier (12) formant un premier segment récepteur de palier d'enroulement (32) et un second segment récepteur de palier d'enroulement (34),
* le palier d'enroulement (30) étant logé de manière amovible dans le premier segment récepteur de palier d'enroulement (32) ou dans le second segment récepteur de palier d'enroulement (34).

11. Dispositif (10) selon la revendication 10,
**caractérisé en ce qu'**il (10) comprend
une manivelle (48) coopérant avec le palier d'enroulement (30) pour tourner les tiges d'enroulement (42).

12. Dispositif (10) selon la revendication 11,
**caractérisé en ce que**
la manivelle (48) est fixée au palier d'enroulement (30) de manière mobile entre une première position et une seconde position.

13. Dispositif (10) selon l'une des revendications 10 à 12,
**caractérisé en ce que**
- le palier d'enroulement (30) a une surface de glissement de palier d'enroulement (38) par laquelle le palier d'enroulement (30) coopère avec le premier segment récepteur de palier d'enroulement (32) et/ou avec le second segment récepteur de palier d'enroulement (34),
- la surface de glissement de palier d'enroulement (38), le premier segment récepteur de palier d'enroulement (32) et/ou le second segment récepteur de palier d'enroulement (34) ayant une surface à faible frottement (40).

14. Dispositif (10) selon l'une des revendications 10 à 13,
**caractérisé en ce que**
- la première moitié de boîtier (14) forme un premier creux (64) tourné vers la cavité (18), et/ou
- la seconde moitié de boîtier (16) forme un second creux (66) tourné vers la cavité (18).

15. Dispositif (10) selon l'une des revendications 10 à 14,
**caractérisé en ce que**
- la première moitié de boîtier (14) forme une première surface extérieure (68) et la seconde moitié de boîtier (16) forme une seconde surface extérieure (70),
- la première surface extérieure (68) et/ou la seconde surface extérieure (70) sont réalisées sous la forme de surfaces extérieures (72) superhydrophobes.
